(19) Europäisches Patentamt
European Patent Office
Office européen des brevets

(11) Veröffentlichungsnummer: **0 529 450 B1**

(12) **EUROPÄISCHE PATENTSCHRIFT**

(45) Veröffentlichungstag der Patentschrift: **21.12.94**

(51) Int. Cl.$^{5}$: **C07D 215/14**, A61K 31/47

(21) Anmeldenummer: **92113956.4**

(22) Anmeldetag: **17.08.92**

Die Akte enthält technische Angaben, die nach dem Eingang der Anmeldung eingereicht wurden und die nicht in dieser Patentschrift enthalten sind.

(54) **Substituierte Mandelsäurederivate, als 5-Lipoxygenasehemmer.**

(30) Priorität: **29.08.91 DE 4128681**

(43) Veröffentlichungstag der Anmeldung:
**03.03.93 Patentblatt 93/09**

(45) Bekanntmachung des Hinweises auf die Patenterteilung:
**21.12.94 Patentblatt 94/51**

(84) Benannte Vertragsstaaten:
**AT BE CH DE DK ES FR GB GR IE IT LI LU MC NL PT SE**

(56) Entgegenhaltungen:
**EP-A- 0 414 078**

(73) Patentinhaber: **BAYER AG**

**D-51368 Leverkusen (DE)**

(72) Erfinder: **Mohrs, Klaus-Helmut, Dr.**
**Wildsteig 24**
**D-5600 Wuppertal (DE)**
Erfinder: **Raddatz, Siegfried, Dr.**
**Jakob-Böhme-Strasse 21**
**D-5000 Köln 80 (DE)**
Erfinder: **Matzke, Michael, Dr.**
**Am Ringelbusch 15**
**D-5600 Wuppertal (DE)**
Erfinder: **Fruchtmann, Romanis**
**Konrad-Adenauer-Weg 79**
**D-5000 Köln (DE)**
Erfinder: **Hatzelmann, Armin, Dr.**
**Alter Wall 3**
**D-7750 Konstanz (DE)**
Erfinder: **Kohlsdorfer, Christian, Dr.**
**Franz-Stryck-Strasse 16**
**D-5042 Erftstadt (DE)**
Erfinder: **Müller-Peddinghaus, Reiner, Prof. Dr.**
**Klutstein 22a**
**D-5060 Bergisch Gladbach 2 (DE)**
Erfinder: **Theisen-Popp, Pia, Dr.**
**Horbacher Strasse 368**
**D-5100 Aachen (DE)**

**EP 0 529 450 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft substituierte Mandelsäurederivate, Verfahren zu ihrer Herstellung und ihre Verwendung in Arzneimitteln.

Es ist bereits bekannt, daß 4-(Chinolin-2-yl-methoxy)phenylessigsäurederivate und $\alpha$-substituierte 4-(Chinolin-2-yl-methoxy)phenylessigsäurederivate eine lipoxygenaseinhibierende Wirkung besitzen [vgl. EP 344 519 (US 4 970 215) und EP 339 416]. Ferner sind (Chinolin-2-yl-methoxy)phenylsubstituierte Mandelsäurederivate mit lipoxygenasehemmender Wirkung aus EP 414078 bekannt.

Die vorliegende Erfindung betrifft substituierte Mandelsäurederivate der allgemeinen Formel (I)

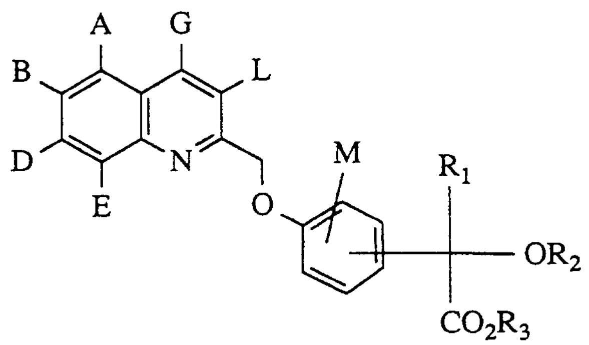

(I),

in welcher

| | |
|---|---|
| A, B, D, E, G, L und M | gleich oder verschieden sind und für Wasserstoff, Hydroxy, Halogen, Trifluormethyl, Trifluormethoxy oder Carboxy stehen, für geradkettiges oder verzweigtes Alkyl mit bis zu 10 Kohlenstoffatomen stehen, das gegebenenfalls durch Hydroxy oder Halogen substituiert ist, für geradkettiges oder verzweigtes Alkoxy oder Alkoxycarbonyl mit bis zu 10 Kohlenstoffatomen stehen, oder für Aryl mit 6 bis 10 Kohlenstoffatomen stehen, das gegebenenfalls durch Halogen, Nitro, Cyano oder durch geradkettiges oder verzweigtes Alkyl oder Alkoxy mit jeweils bis zu 8 Kohlenstoffatomen substituiert ist, |
| $R^1$ | für eine Gruppe der Formel |

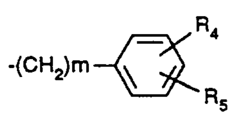

,

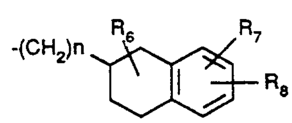

oder

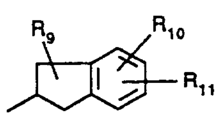

| | |
|---|---|
| | steht, worin |
| n und m | gleich oder verschieden sind und eine Zahl 1, 2, 3, 4, 5, 6, 7 oder 8 bedeuten, |
| $R^4$, $R^5$, $R^6$, $R^7$, $R^8$, $R^9$, $R^{10}$ und $R^{11}$ | gleich oder verschieden sind und Wasserstoff, Halogen, Nitro, Cyano, Hydroxy, Carboxy, Trifluormethyl, Trifluormethoxy oder geradkettiges oder verzweigtes Alkyl, Alkoxy oder Alkoxycarbonyl mit jeweils bis zu 8 Kohlenstoffatomen bedeuten, |
| $R^2$ | für Wasserstoff oder für geradkettiges oder verzweigtes Alkyl mit bis zu 8 Kohlenstoffatomen steht, |
| $R^3$ | für Wasserstoff, geradkettiges oder verzweigtes Alkyl mit bis zu 8 Kohlenstoffatomen steht |

und deren Salze.

Im Rahmen der vorliegenden Erfindung werden physiologisch unbedenkliche Salze bevorzugt. Physiologisch unbedenkliche Salze der substituierten Mandelsäure-Derivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Metallsalze, bevorzugt der einwertigen Metalle, und die Ammoniumsalze. Bevorzugt werden Alkalisalze wie beispielsweise Natrium-, Kalium- und Ammoniumsalze.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

A, B, D, E, G, L und M gleich oder verschieden sind und für Wasserstoff, Fluor, Chlor, Brom, Trifluormethoxy oder Carboxy stehen, für geradkettiges oder verzweigtes Alkyl mit bis zu 8 Kohlenstoffatomen stehen, das gegebenenfalls durch Hydroxy, Fluor, Chlor oder Brom substituiert ist,
für geradkettiges oder verzweigtes Alkoxy oder Alkoxycarbonyl mit jeweils bis zu 8 Kohlenstoffatomen stehen, oder
für Phenyl stehen, das gegebenenfalls durch Fluor, Chlor, Brom, Nitro, Cyano oder durch geradkettiges oder verzweigtes Alkyl oder Alkoxy mit jeweils bis zu 6 Kohlenstoffatomen substituiert ist,

$R^1$ für eine Gruppe der Formel

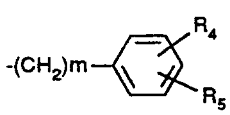 , 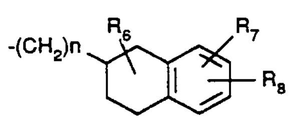 oder 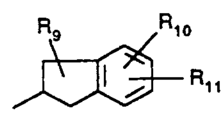

steht,
worin

n und m gleich oder verschieden sind und eine Zahl 1, 2, 3, 4, 5, 6 oder 7 bedeuten,

$R^4$, $R^5$, $R^6$, $R^7$, $R^8$, $R^9$, $R^{10}$ und $R^{11}$ gleich oder verschieden sind und Wasserstoff, Fluor, Chlor, Brom, Trifluormethyl, Trifluormethoxy oder geradkettiges oder verzweigtes Alkyl oder Alkoxy mit jeweils bis zu 6 Kohlenstoffatomen bedeuten,

$R^2$ für Wasserstoff oder geradkettiges oder verzweigtes Alkyl mit bis zu 6 Kohlenstoffatomen steht,

$R^3$ für Wasserstoff, geradkettiges oder verzweigtes Alkyl mit bis zu 6 Kohlenstoffatomen steht

und deren Salze.

Besonders bevorzugt sind Verbindungen der allgemeinen Formel (I),
in welcher

A, B, D, E, G, L und M gleich oder verschieden sind und für Wasserstoff, Fluor, Chlor, geradkettiges oder verzweigtes Alkyl oder Alkoxy mit jeweils bis zu 6 Kohlenstoffatomen stehen,

$R^1$ für eine Gruppe der Formel

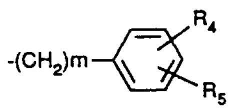 , 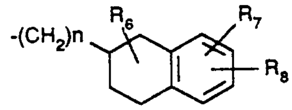 oder 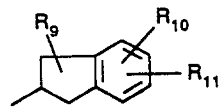

steht,
worin

n und m gleich oder verschieden sind und eine Zahl 1, 2, 3, 4, 5 oder 6 bedeuten,

$R^4$, $R^5$, $R^6$, $R^7$, $R^8$, $R^9$, $R^{10}$ und $R^{11}$ gleich oder verschieden sind und Wasserstoff, Fluor, Chlor, Brom, Trifluormethyl, Trifluormethoxy oder geradkettiges oder verzweigtes Alkyl oder Alkoxy mit jeweils bis zu 4 Kohlenstoffatomen bedeuten,

$R^2$ für Wasserstoff oder geradkettiges oder verzweigtes Alkyl mit bis zu 4 Kohlenstoffatomen steht,

$R^3$ für Wasserstoff, geradkettiges oder verzweigtes Alkyl mit bis zu 4 Kohlenstoffatomen steht

und deren Salze.

Ganz besonders bevorzugt sind Verbindungen der allgemeinen Formel (I),
in welcher
A, B, D, E, G, L und M für Wasserstoff stehen. Ferner sind solche Verbindungen besonders bevorzugt, bei denen der Rest der Formel $-C(R^1)(OR^2)(CO_2R^3)$ in 4-Stellung zum Chinolylmethoxyrest steht.

Außerdem wurde ein Verfahren zur Herstellung der erfindungsgemäßen Verbindungen der allgemeinen Formel (I) gefunden,
dadurch gekennzeichnet, daß man Glyoxylester der allgemeinen Formel (II)

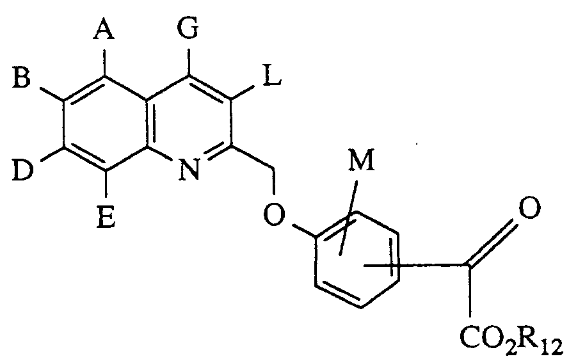

(II),

in welcher

A, B, D, E, G, L und M die oben angegebene Bedeutung haben

und

$R^{12}$ die oben angegebene Bedeutung von $R^3$ hat, aber nicht für Wasserstoff steht mit Grignard- oder metallorganischen Verbindungen der allgemeinen Formel (III)

$R^1$-V (III)

in welcher

$R^1$ die oben angegebene Bedeutung hat

und

V für den typischen Grignardrest W-Z steht,
worin

W Magnesium, Cadmium oder Zink bedeutet
und

Z Chlor, Brom oder Jod bedeutet,

oder

für Lithium, Natrium, Magnesium, Aluminium, Cadmium oder Zink steht,

in inerten Lösemitteln unter Abspaltung der Gruppe V reduziert,

und im Fall, daß $R^2$ nicht für Wasserstoff steht, nach üblicher Methode verethert, und im Fall der Säuren ($R^3$ = H) die Ester verseift,

und im Fall der Enantiomeren die entsprechenden enantiomerenreinen Säuren ($R^3$ = H) trennt, wobei die Substituenten A, B, D, E, G, L und M gegebenenfalls nach üblichen Methoden variiert werden können.

Das erfindungsgemäße Verfahren kann durch Formelschema beispielhaft erläutert werden:

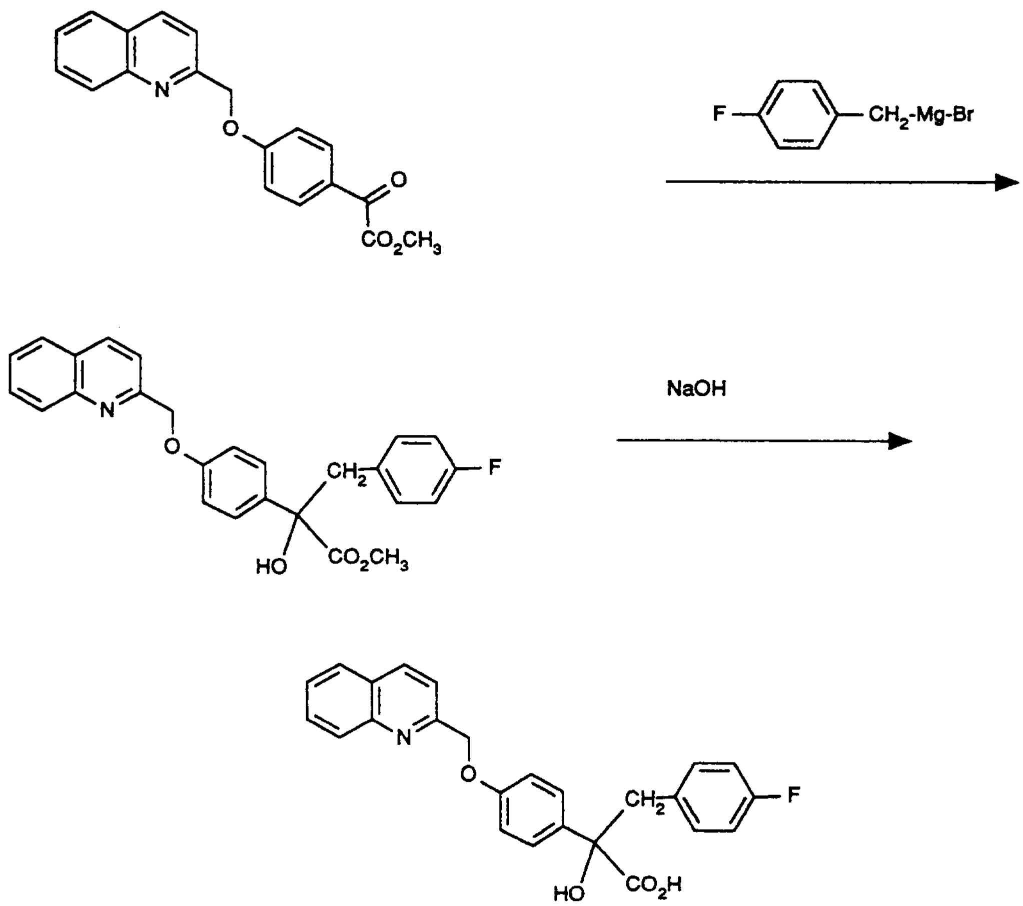


Als Lösemittel für die Reduktion eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan, oder Erdölfraktionen oder Dimethylformamid. Ebenso ist es möglich, Gemische der genannten Lösemittel zu verwenden. Bevorzugt sind Tetrahydrofüran und Diethylether.

Die Reduktion wird im allgemeinen in einem Temperaturbereich von -80 °C bis +30 °C, bevorzugt bei -40 °C bis +25 °C durchgeführt.

Die Reduktion wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Gruppe V erfolgt nach der für Grignard-Reaktionen üblichen Methode mit wäßriger Ammoniumchloridlösung [vgl. J. March, Advanced Organic Chemistry, Second Edition S. 836].

Die Verbindungen der allgemeinen Formeln (III) sind an sich bekannt oder können nach üblicher Methode hergestellt werden [vgl. K. Nützel, Houben-Weyl, Methoden der organischen Chemie, 4. Aufl. Bd. 13/2a, 53 ff. (Thieme Verlag, Stuttgart) 1973; M.S. Kharash, O. Reinmuth, Grignard Reactions of Nonmetallic Compounds, Prentice Hall, New York, 1974; Uhlman XII, 370; Houben-Weyl XIII/2a, 289-302; R.I. Trust, R.E. Ireland, Org. Synth. 53, 116, (1973); O. Grummitt, E.I. Becker, Org. Synth. Coll. Vol. IV, 771 (1963); H. Adkins, W. Zartman, Org. Synth. Coll. Vol. II, 606 (1943)].

Im allgemeinen setzt man 1 bis 3 Mol, bevorzugt 1,1 Mol der Grignard Verbindungen bzw. der metallorganischen Verbindungen der allgemeinen Formeln (III) bezogen auf 1 Mol der Glyoxylester der allgemeinen Formel (II) ein.

Die Verbindungen der allgemeinen Formel (II) sind an sich bekannt [vgl. EP 414 078] und können beispielsweise hergestellt werden, indem man
Verbindungen der allgemeinen Formel (IV)

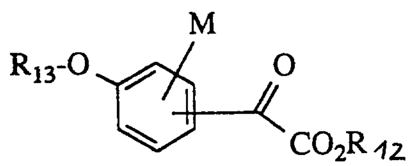

(IV)

in welcher

$R^{12}$ und M die oben angegebene Bedeutung haben

und

$R^{13}$ für eine typische Hydroxyschutzgruppe, wie beispielsweise Benzyl oder tert.Butyl steht,

mit Halogenmethylchinolinen der Formel (V)

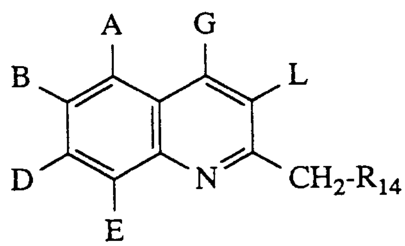

(V)

in welcher

A, B, D, E, G und L die oben angegebene Bedeutung haben

und

$R^{14}$ für Halogen, vorzugsweise für Chlor oder Brom steht,

nach Abspaltung der Schutzgruppe $R^{13}$ in inerten Lösemitteln, gegebenenfalls in Anwesenheit einer Base verethert.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley / Sons, 1981, New York].

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0 °C bis +150 °C, bevorzugt von +10 °C bis +100 °C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol des Reaktionspartners ein. Die Base wird im allgemeinen in einer Menge von 0,5 bis 5 Mol, bevorzugt von 1 bis 3 Mol bezogen auf das Halogenid eingesetzt.

Die Verbindungen der allgemeinen Formel (IV) sind bekannt oder können nach üblicher Methode hergestellt werden [vgl. Chem. Commun. 1972, (11), 668].

Die Verbindungen der allgemeinen Formel (V) sind ebenfalls bekannt oder können nach üblicher Methode hergestellt werden [vgl. Chem. Ber. 120, 649 (1987)].

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofüran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt

Im allgemeinen wird die Verseifung bei Normaldruck durchgeführt Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Verbindungen der allgemeinen Formel (I) zeigen überraschenderweise eine hohe Aktivität als Leukotriensynthesehemmer insbesondere nach oraler Applikation.

Die erfindungsgemäßen substituierten Mandelsäurederivate können als Wirkstoffe in Arzneimitteln eingesetzt werden. Die Stoffe können als Hemmer von enzymatischen Reaktionen im Rahmen des Arachldonsäurestoffwechsels, insbesondere der 5-Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen substituierten Mandelsäurederivate können sowohl in der Humanmedizin als auch in der Veterinärmedizin angewendet werden.

Die pharmakologischen Wirkdaten der erfindungsgemäßen Substanzen werden durch folgende Methode bestimmt:

Als Maß für die 5-Lipoxygenase-Hemmung wurde die Freisetzung von Leukotrien $B_4$ ($LTB_4$) an polymorphkernigen Humanleukozyten (PMN) nach Zugabe von Substanzen und Ca-lonophor mittels reverse phase HPLC nach Borgeat, P. et al., Proc. Nat. Acad. Sci. 76, 2148-2152 (1979), bestimmt.

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50

mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Herstellungsbeispiele

Beispiel 1

2-[4-(Chinolin-2-yl-methoxy)phenyl]-3-(4-fluorphenyl)-2-hydroxy-propionsäuremethylester

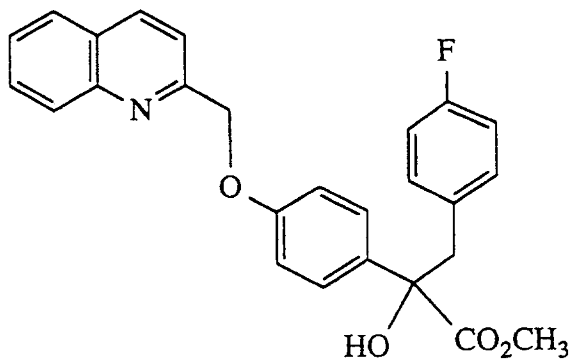

Zu einer Lösung von 5 g (0,0158 mol) 4-(Chinolin-2-yl-methoxy)phenylglyoxylsäuremethylester (Herstellung: Mohrs et al., EP 414 078 A2) in 50 ml Tetrahydrofuran wird unter Schutzgas und Feuchtigkeitsauschluß bei 0 °C eine frisch bereitete Grignard-Lösung aus 4,86 g (0,0257 mol) 4-Fluorbenzylbromid, 0,625 g (0,0257 mol) Magnesiumspänen in 50 ml Diethylether langsam zugetropft. Nach Erwärmen auf 25 °C wird das Reaktionsgemisch auf Eiswasser gegossen, mit Ammoniumchlorid angesäuert zweimal mit Essigester extrahiert, die organischen Phasen über Natriumsulfat getrocknet, eingedampft und der Rückstand an Kieselgel 60 (Cyclohexan / Essigester 3:1) chromatographiert.

Ausbeute: 2,17g (31,8% der Theorie)

Fp.: 155 °C($H_3COH$)

Analog Beispiel 1 werden die in Tabelle 1 aufgeführten Verbindungen hergestellt:

Tabelle 1:

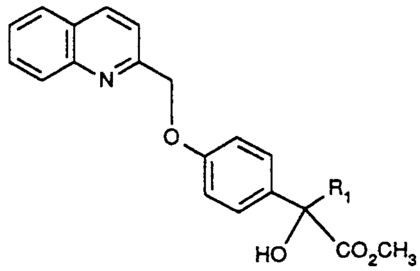

| Bsp.-Nr. | $R^1$ | Fp.: | Ausbeute |
|---|---|---|---|
| 2 | 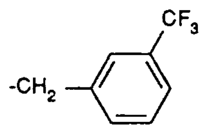 | 89°C | 48,6% |
| 3 | 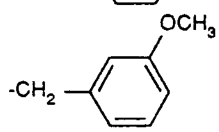 | - a) | 83% |
| 4 | 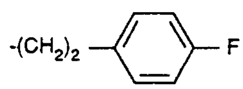 | - a) | 30,5% |
| 5 | 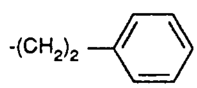 | - a) | 32,2% |
| 6 | 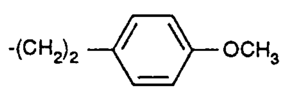 | - a) | 30,4% |
| 7 | 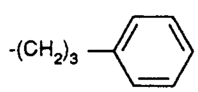 | - a) | 43,3% |
| 8 | 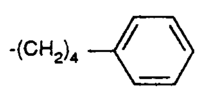 | - a) | 37,5% |
| 9 | 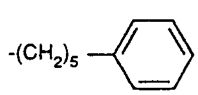 | - a) | 40,0% |
| 10 | 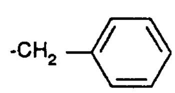 | 161°C | 23,0% |

Fortsetzung Tabelle 1:

| Bsp.-Nr. | $R^1$ | Fp.: | Ausbeute |
|---|---|---|---|
| 11 | 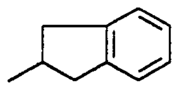 | 132°C | 30,7% |
| 12 | 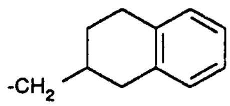 | - a) | 36,5% |

a) Die Verbindungen werden nach Chromatographie sofort weiter umgesetzt.

Beispiel 13

2-[4-(Chinolin-2-yl-methoxy)phenyl]-3-(4-fluorphenyl)-2-hydroxy-propionsäure

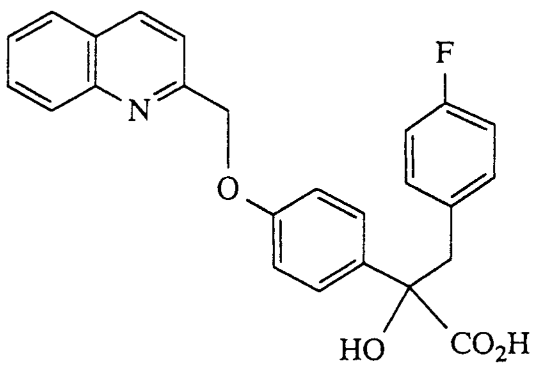

2,1 g (48,7 mmol) der Verbindung aus Beispiel 1 werden in 50 ml Methanol und 5 ml 2 N Natronlauge 15 h unter Rückfluß erwärmt. Nach Abkühlen wird mit 5 ml 2 N Salzsäure neutralisiert, das ausgefallene Produkt abgesaugt und aus Methanol umkristallisiert.
Ausbeute: 1,86 g (91,6% der Theorie)
Festpunkt: 203 °C ($H_3COH$)
Analog zu Beispiel 13 werden die in Tabelle 2 aufgeführten Derivate hergestellt:

Tabelle 2:

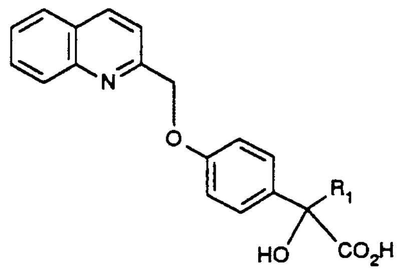

| Bsp.-Nr. | $R^1$ | Fp.: b) | Ausbeute |
|---|---|---|---|
| 14 | 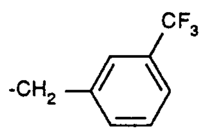 | 204°C | 57% |
| 15 | 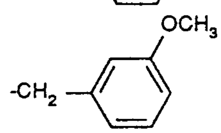 | 228°C | 78,5% |
| 16 | 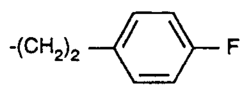 | 168°C | 51% |
| 17 | 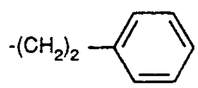 | 178°C | 67% |
| 18 | 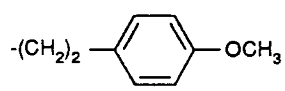 | 182°C | 67,7% |
| 19 | 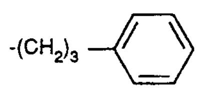 | 194°C | 61% |
| 20 | 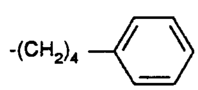 | 147°C | 73,7% |
| 21 | 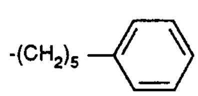 | 141°C | 52% |
| 22 | 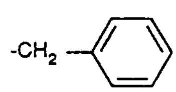 | 200°C | 74,5% |

Fortsetzung Tabelle 2:

| Bsp.-Nr. | $R^1$ | Fp.: [b)] | Ausbeute |
|---|---|---|---|
| 23 | 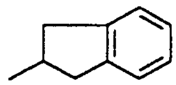 | 208°C | 68,5% |
| 24 | 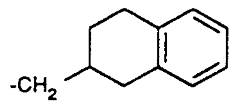 | 194°C | 88,2% |

b) umkristallisiert aus Methanol

Beispiele 25 bis 26

(+)-2-[4-(Chinolin-2-yl-methoxy)phenyl]-2-(2-indanyl)-2-hydroxyessigsäure (25)

(-)-2-[4-(Chinolin-2-yl-methoxy)phenyl]-2-(2-indanyl)-2-hydroxyessigsäure (26)

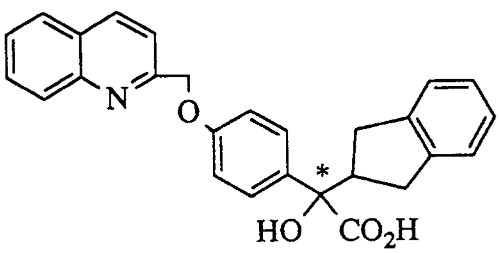

5 g des Racemates aus Beispiel 23 wurden an chiralen Phasen unter Standardbedingungen präparativ getrennt.
Ausbeute je 2 g der enantiomerenreinen Verbindungen.

| | |
|---|---|
| (+) Enantiomer: (25) | ee > 99 (HPLC)<br>$\alpha_D^{20}$ + 18,5 (c = 1, MeOH)<br>Festpunkt: 181 ° C (MeOH) |

| | |
|---|---|
| (-) Enantiomer: (26) | ee > 99 (HPLC)<br>$\alpha_D^{20}$ - 18,8 (c = 1, MeOH)<br>Festpunkt: 181 ° C (MeOH) |

**Patentansprüche**

**Patentansprüche für folgende Vertragsstaaten : AT, BE, CH, DE, DK, FR, GB, GR, IE, IT, LI, LU, MC, NL, PT, SE**

**1.** Substituierte Mandelsäurederivate der allgemeinen Formel (I)

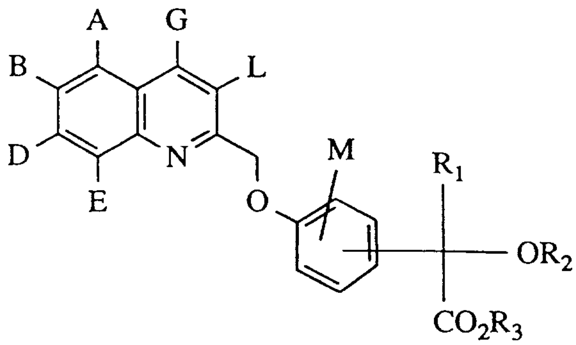

(I),

in welcher

| | |
|---|---|
| A, B, D, E, G, L und M | gleich oder verschieden sind und für Wasserstoff, Hydroxy, Halogen, Trifluormethyl, Trifluormethoxy oder Carboxy stehen, für geradkettiges oder verzweigtes Alkyl mit bis zu 10 Kohlenstoffatomen stehen, das gegebenenfalls durch Hydroxy oder Halogen substituiert ist, für geradkettiges oder verzweigtes Alkoxy oder Alkoxycarbonyl mit bis zu 10 Kohlenstoffatomen stehen, oder für Aryl mit 6 bis 10 Kohlenstoffatomen stehen, das gegebenenfalls durch Halogen, Nitro, Cyano oder durch geradkettiges oder verzweigtes Alkyl oder Alkoxy mit jeweils bis zu 8 Kohlenstoffatomen substituiert ist, |
| $R^1$ | für eine Gruppe der Formel |

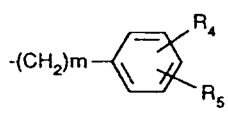

,

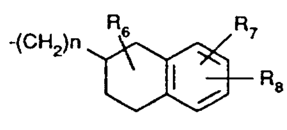

oder

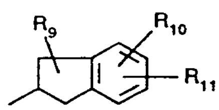

| | |
|---|---|
| | steht, worin |
| n und m | gleich oder verschieden sind und eine Zahl 1, 2, 3, 4, 5, 6, 7 oder 8 bedeuten, |
| $R^4$, $R^5$, $R^6$, $R^7$, $R^8$, $R^9$, $R^{10}$ und $R^{11}$ | gleich oder verschieden sind und Wasserstoff, Halogen, Nitro, Cyano, Hydroxy, Carboxy, Trifluormethyl, Trifluormethoxy oder geradkettiges oder verzweigtes Alkyl, Alkoxy oder Alkoxycarbonyl mit jeweils bis zu 8 Kohlenstoffatomen bedeuten, |
| $R^2$ | für Wasserstoff oder für geradkettiges oder verzweigtes Alkyl mit bis zu 8 Kohlenstoffatomen steht, |
| $R^3$ | für Wasserstoff, geradkettiges oder verzweigtes Alkyl mit bis zu 8 Kohlenstoffatomen steht |

und deren Salze.

**2.** Substituierte Mandelsäurederivate nach Anspruch 1, wobei

| | |
|---|---|
| A, B, D, E, G, L und M | gleich oder verschieden sind und |

für Wasserstoff, Fluor, Chlor, Brom, Trifluormethoxy oder Carboxy stehen,
für geradkettiges oder verzweigtes Alkyl mit bis zu 8 Kohlenstoffatomen stehen, das gegebenenfalls durch Hydroxy, Fluor, Chlor oder Brom substituiert ist,
für geradkettiges oder verzweigtes Alkoxy oder Alkoxycarbonyl mit jeweils bis zu 8 Kohlenstoffatomen stehen, oder
für Phenyl stehen, das gegebenenfalls durch Fluor; Chlor, Brom, Nitro, Cyano oder durch geradkettiges oder verzweigtes Alkyl oder Alkoxy mit jeweils bis zu 6 Kohlenstoffatomen substituiert ist,

$R^1$ für eine Gruppe der Formel

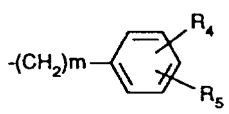
,
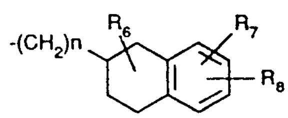
oder
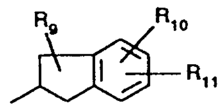

steht,
worin

n und m gleich oder verschieden sind und eine Zahl 1, 2, 3, 4, 5, 6 oder 7 bedeuten,

$R^4$, $R^5$, $R^6$, $R^7$, $R^8$, $R^9$, $R^{10}$ und $R^{11}$ gleich oder verschieden sind und Wasserstoff, Fluor, Chlor, Brom, Trifluormethyl, Trifluormethoxy oder geradkettiges oder verzweigtes Alkyl oder Alkoxy mit jeweils bis zu 6 Kohlenstoffatomen bedeuten,

$R^2$ für Wasserstoff oder geradkettiges oder verzweigtes Alkyl mit bis zu 6 Kohlenstoffatomen steht,

$R^3$ für Wasserstoff, geradkettiges oder verzweigtes Alkyl mit bis zu 6 Kohlenstoffatomen steht

und deren Salze.

**3.** Substituierte Mandelsäurederivate nach Anspruch 1,
wobei

A, B, D, E, G, L und M gleich oder verschieden sind und
für Wasserstoff, Fluor, Chlor, geradkettiges oder verzweigtes Alkyl oder Alkoxy mit jeweils bis zu 6 Kohlenstoffatomen stehen,

$R^1$ für eine Gruppe der Formel

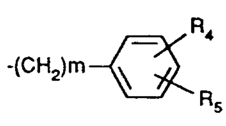
,
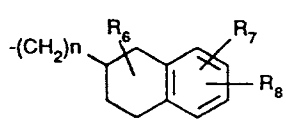
oder
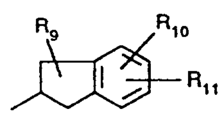

steht,
worin

n und m gleich oder verschieden sind und eine Zahl 1, 2, 3, 4, 5 oder 6 bedeuten,

$R^4$, $R^5$, $R^6$, $R^7$, $R^8$, $R^9$, $R^{10}$ und $R^{11}$ gleich oder verschieden sind und Wasserstoff, Fluor, Chlor, Brom, Trifluormethyl, Trifluormethoxy oder geradkettiges oder verzweigtes Alkyl oder Alkoxy mit jeweils bis zu 4 Kohlenstoffatomen bedeuten,

$R^2$ für Wasserstoff oder geradkettiges oder verzweigtes Alkyl mit bis zu 4 Kohlenstoffatomen steht,

$R^3$ für Wasserstoff, geradkettiges oder verzweigtes Alkyl mit bis

zu 4 Kohlenstoffatomen steht

und deren Salze.

**4.** Substituierte Mandelsäurederivate nach Anspruch 1 worin der Rest der Formel $-C(R^1)(OR^2)(CO_2R^3)$ in 4-Stellung zum Chinolylmethoxyrest steht.

**5.** Alpha-[4-(2-Chinolinyl-methoxy)-phenyl]-alpha-hydroxy-2-indanessigsäure der Formel

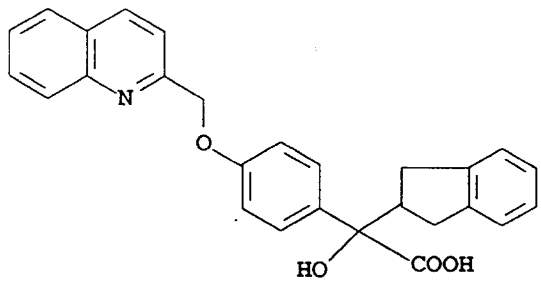

dessen Stereoisomere und dessen Salze.

**6.** Substituierte Mandelsäurederivate nach Anspruch 1 - 5 zur Behandlung von Krankheiten.

**7.** Verfahren zur Herstellung von substituierten Mandelsäurederivaten nach Anspruch 1 - 5, dadurch gekennzeichnet, daß man Glyoxylester der allgemeinen Formel (II)

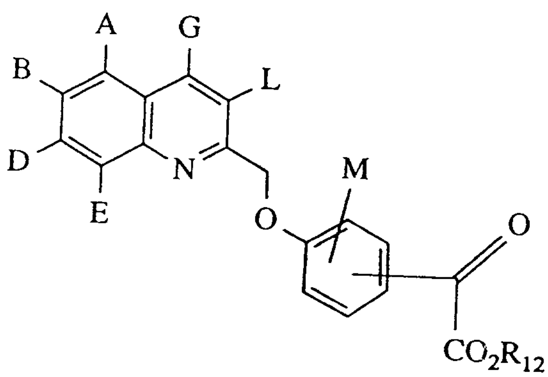

(II),

in welcher

A, B, D, E, G, L und M die oben angegebene Bedeutung haben

und

$R^{12}$ die oben angegebene Bedeutung von $R^3$ hat, aber nicht für Wasserstoff steht

mit Grignard- oder metallorganischen Verbindungen der allgemeinen Formel (III)

$R^1$-V (III)

in welcher

$R^1$ die oben angegebene Bedeutung hat

und

V für den typischen Grignardrest W-Z steht, worin

W Magnesium, Cadmium oder Zink bedeutet

und

Z Chlor, Brom oder Jod bedeutet,

oder

für Lithium, Natrium, Magnesium, Aluminium, Cadmium oder Zink steht,

in inerten Lösemitteln unter Abspaltung der Gruppe V reduziert,
und im Fall, daß $R^2$ nicht für Wasserstoff steht, nach üblicher Methode verethert,
und im Fall der Säuren ($R^3$ = H) die Ester verseift,
und im Fall der Enantiomeren die entsprechenden enantiomerenreinen Säuren $R^3$ = H nach den üblichen Methoden trennt, wobei die Substituenten A, B, D, E, G, L und M gegebenenfalls nach üblichen Methoden variiert werden können.

**8.** Arzneimittel enthaltend substituierte Mandelsäurederivate nach Anspruch 1 - 5.

**9.** Verwendung von substituierten Mandelsäurederivaten nach Anspruch 1 - 5 zur Herstellung von Arzneimitteln.

**10.** Verwendung von substituierten Mandelsäurederivaten nach Anspruch 1 - 5 zur Herstellung von Leukotriensynthese-Hemmern.

**Patentanspruch für folgenden Vertragsstaat : ES**

**1.** Verfahren zur Herstellung von substituierten Mandelsäurederivaten der allgemeinen Formel (I)

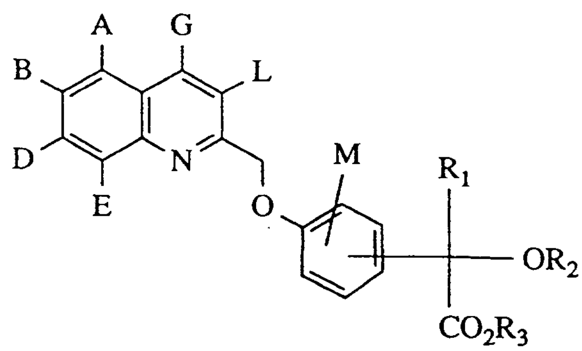

(I),

in welcher

| | |
|---|---|
| A, B, D, E, G, L und M | gleich oder verschieden sind und für Wasserstoff, Hydroxy, Halogen, Trifluormethyl, Trifluormethoxy oder Carboxy stehen, für geradkettiges oder verzweigtes Alkyl mit bis zu 10 Kohlenstoffatomen stehen, das gegebenenfalls durch Hydroxy oder Halogen substituiert ist, für geradkettiges oder verzweigtes Alkoxy oder Alkoxycarbonyl mit bis zu 10 Kohlenstoffatomen stehen, oder für Aryl mit 6 bis 10 Kohlenstoffatomen stehen, das gegebenenfalls durch Halogen, Nitro, Cyano oder durch geradkettiges oder verzweigtes Alkyl oder Alkoxy mit jeweils bis zu 8 Kohlenstoffatomen substituiert ist, |
| $R^1$ | für eine Gruppe der Formel |

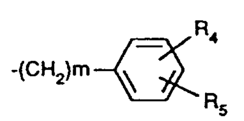

,

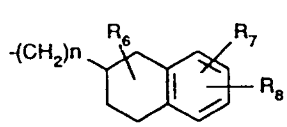

oder

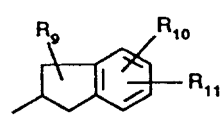

| | |
|---|---|
| | steht, worin |
| n und m | gleich oder verschieden sind und eine Zahl 1, 2, 3, 4, 5, 6, 7 oder 8 bedeuten, |
| $R^4$, $R^5$, $R^6$, $R^7$, $R^8$, $R^9$, $R^{10}$ und $R^{11}$ | gleich oder verschieden sind und Wasserstoff, Halogen, Ni- |

tro, Cyano, Hydroxy, Carboxy, Trifluormethyl, Trifluormethoxy oder geradkettiges oder verzweigtes Alkyl, Alkoxy oder Alkoxycarbonyl mit jeweils bis zu 8 Kohlenstoffatomen bedeuten,

$R^2$ für Wasserstoff oder für geradkettiges oder verzweigtes Alkyl mit bis zu 8 Kohlenstoffatomen steht,

$R^3$ für Wasserstoff, geradkettiges oder verzweigtes Alkyl mit bis zu 8 Kohlenstoffatomen steht

und deren Salze,

dadurch gekennzeichnet, daß man Glyoxylester der allgemeinen Formel (II)

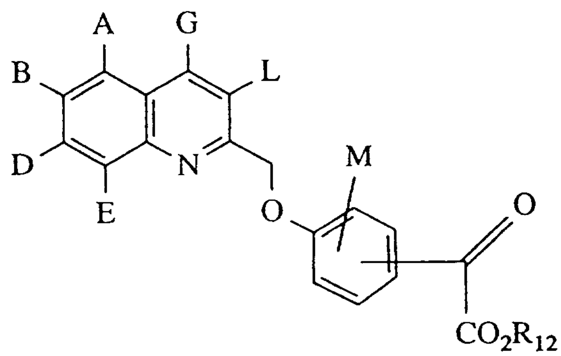

(II),

in welcher

A, B, D, E, G, L und M die oben angegebene Bedeutung haben

und

$R^{12}$ die oben angegebene Bedeutung von $R^3$ hat, aber nicht für Wasserstoff steht

mit Grignard- oder metallorganischen Verbindungen der allgemeinen Formel (III)

$R^1$-V (III)

in welcher

$R^1$ die oben angegebene Bedeutung hat

und

V für den typischen Grignardrest W-Z steht, worin

W Magnesium, Cadmium oder Zink bedeutet und

Z Chlor, Brom oder Jod bedeutet,

oder

für Lithium, Natrium, Magnesium, Aluminium, Cadmium oder Zink steht,

in inerten Lösemitteln unter Abspaltung der Gruppe V reduziert,

und im Fall, daß $R^2$ nicht für Wasserstoff steht, nach üblicher Methode verethert,

und im Fall der Säuren ($R^3$ = H) die Ester verseift,

und im Fall der Enantiomeren die entsprechenden enantiomerenreinen Säuren $R^3$ = H nach den üblichen Methoden trennt, wobei die Substituenten A, B, D, E, G, L und M gegebenenfalls nach üblichen Methoden variiert werden können.

**Claims**

**Claims for the following Contracting States : AT, BE, CH, DE, DK, FR, GB, GR, IE, IT, LI, LU, MC, NL, PT, SE**

**1.** Substituted mandelic acid derivatives of the general formula (I)

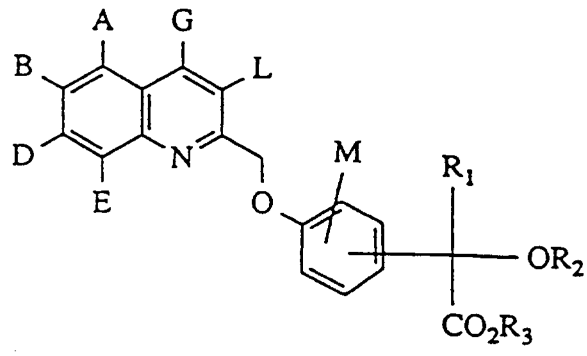

(I)

in which

| | |
|---|---|
| A, B, D, E, G, L and M | are identical or different and represent hydrogen, hydroxyl, halogen, trifluoromethyl, trifluoromethoxy or carboxyl, represent straight-chain or branched alkyl which has up to 10 carbon atoms and is optionally substituted by hydroxyl or halogen, represent straight-chain or branched alkoxy or alkoxycarbonyl having up to 10 carbon atoms or represent aryl which has 6 to 10 carbon atoms and is optionally substituted by halogen, nitro, cyano or by straight-chain or branched alkyl or alkoxy having in each case up to 8 carbon atoms, |
| $R^1$ | represents a group of the formula |

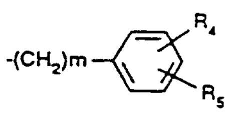

, 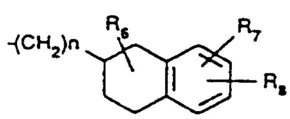 or 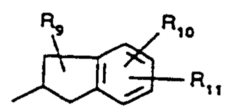

| | |
|---|---|
| | wherein |
| n and m | are identical or different and denote the number 1, 2, 3, 4, 5, 6, 7 or 8 and |
| $R^4$, $R^5$, $R^6$, $R^7$, $R^8$, $R^9$, $R^{10}$ and $R^{11}$ | are identical or different and denote hydrogen, halogen, nitro, cyano, hydroxyl, carboxyl, trifluoromethyl, trifluoromethoxy or straight-chain or branched alkyl, alkoxy or alkoxycarbonyl having in each case up to 8 carbon atoms, |
| $R^2$ | represents hydrogen or represents straight-chain or branched alkyl having up to 8 carbon atoms, and |
| $R^3$ | represents hydrogen or straight-chain or branched alkyl having up to 8 carbon atoms, |

and salts thereof.

**2.** Substituted mandelic acid derivatives according to Claim 1, wherein

| | |
|---|---|
| A, B, D, E, G, L and M | are identical or different and represent hydrogen, fluorine, chlorine, bromine, trifluoromethoxy or carboxyl, represent straight-chain or branched alkyl which has up to 8 carbon atoms and is optionally substituted by hydroxyl, flu- |

orine, chlorine or bromine,
represent straight-chain or branched alkoxy or alkoxycarbonyl having in each case up to 8 carbon atoms or
represent phenyl which is optionally substituted by fluorine, chlorine, bromine, nitro, cyano or by straight-chain or branched alkyl or alkoxy having in each case up to 6 carbon atoms,

$R^1$ represents a group of the formula

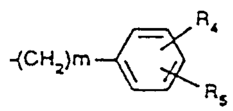 , 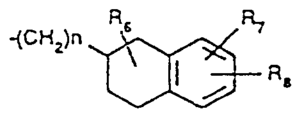 or 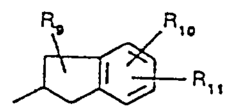

wherein

n and m are identical or different and denote the number 1, 2, 3, 4, 5, 6 or 7 and

$R^4$, $R^5$, $R^6$, $R^7$, $R^8$, $R^9$, $R^{10}$ and $R^{11}$ are identical or different and denote hydrogen, fluorine, chlorine, bromine, trifluoromethyl, trifluoromethoxy or straight-chain or branche d alkyl or alkoxy having in each case up to 6 carbon atoms,

$R^2$ represents hydrogen or straight-chain or branched alkyl having up to 6 carbon atoms and

$R^3$ represents hydrogen or straight-chain or branched alkyl having up to 6 carbon atoms,

and salts thereof.

**3.** Substituted mandelic acid derivatives according to Claim 1, wherein

A, B, D, E, G, L and M are identical or different and represent hydrogen, fluorine, chlorine or straight-chain or branched alkyl or alkoxy having in each case up to 6 carbon atoms,

$R^1$ represents a group of the formula

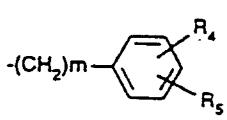 , 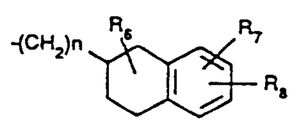 or 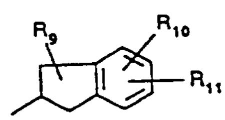

wherein

n and m are identical or different and denote the number 1, 2, 3, 4, 5 or 6 and

$R^4$, $R^5$, $R^6$, $R^7$, $R^8$, $R^9$, $R^{10}$ and $R^{11}$ are identical or different and denote hydrogen, fluorine, chlorine, bromine, trifluoromethyl, trifluoromethoxy or straight-chain or branched alkyl or alkoxy having in each case up to 4 carbon atoms,

$R^2$ represents hydrogen or straight-chain or branched alkyl having up to 4 carbon atoms and

$R^3$ represents hydrogen or straight-chain or branched alkyl having up to 4 carbon atoms,

and salts thereof.

**4.** Substituted mandelic acid derivatives according to Claim 1, wherein the radical of the formula $-C(R^1)-(OR^2)$ is in the 4-position relative to the quinolylmethoxy radical.

5. Alpha-[4-(2-quinolinyl-methoxy)-phenyl]-alpha-hydroxy-2-indaneacetic acid of the formula

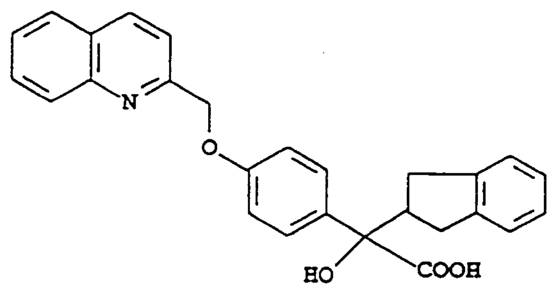

stereoisomers and salts thereof.

6. Substituted mandelic acid derivatives according to Claims 1 - 5, for the treatment of diseases.

7. Process for the preparation of substituted mandelic acid derivatives according to Claims 1 - 5, characterized in that glyoxylic esters of the general formula (II)

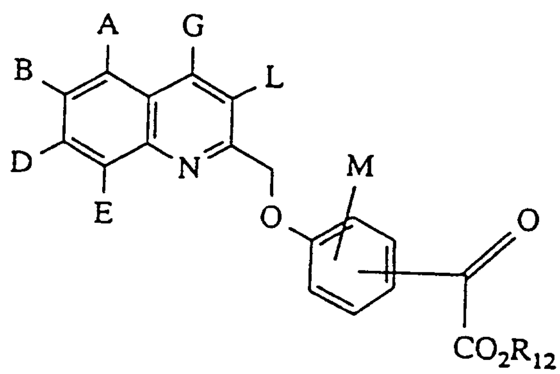

(II)

in which
A, B, D, E, G, L and M have the abovementioned meaning
and
$R^{12}$ has the abovementioned meaning of $R^3$, but does not represent hydrogen,
are reduced with Grignard or organometallic compounds of the general formula (III)

$R^1$-V (III)

in which
$R^1$ has the abovementioned meaning
and
V represents the typical Grignard radical W-Z,
wherein
W denotes magnesium, cadmium or zinc
and
Z denotes chlorine, bromine or iodine,
or
represents lithium, sodium, magnesium, aluminium, cadmium or zinc,
in inert solvents, the group V being split off,
and in the case where $R^2$ does not represent hydrogen, the products are etherified by the customary method,
and in the case of the acids ($R^3$ = H), the eaters are hydrolysed,
and in the case of the enantiomers, the corresponding enantiomerically pure acids $R^3$ = H are separated by the customary methods, it being possible for the substituents A, B, D, E, G, L and M to be varied by customary methods, if appropriate.

8. Medicaments containing substituted mandelic acid derivatives according to Claims 1 - 5.

9. Use of substituted mandelic acid derivatives according to Claims 1 - 5 for the preparation of medicaments.

10. Use of substituted mandelic acid derivatives according to Claims 1 - 5 for the preparation of inhibitors of leukotriene synthesis.

**Claim for the following Contracting State : ES**

1. Process for the preparation of substituted mandelic acid derivatives of the general formula (I)

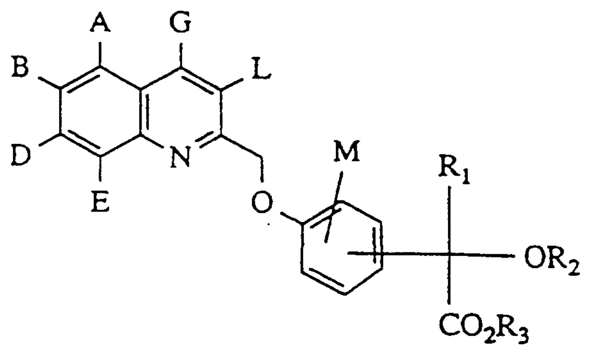

(I)

in which

| | |
|---|---|
| A, B, D, E, G, L and M | are identical or different and represent hydrogen, hydroxyl, halogen, trifluoromethyl, trifluoromethoxy or carboxyl, represent straight-chain or branched alkyl which has up to 10 carbon atoms and is optionally substituted by hydroxyl or halogen,<br>represent straight-chain or branched alkoxy or alkoxycarbonyl having up to 10 carbon atoms or represent aryl which has 6 to 10 carbon atoms and is optionally substituted by halogen, nitro, cyano or by straight-chain or branched alkyl or alkoxy having in each case up to 8 carbon atoms, |
| $R^1$ | represents a group of the formula |

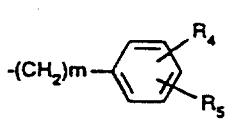

,

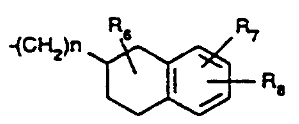

or

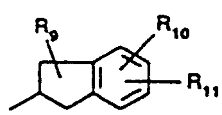

| | |
|---|---|
| | wherein |
| n and m | are identical or different and denote the number 1, 2, 3, 4, 5, 6, 7 or 8 and |
| $R^4$, $R^5$, $R^6$, $R^7$, $R^8$, $R^9$, $R^{10}$ and $R^{11}$ | are identical or different and denote hydrogen, halogen, nitro, cyano, hydroxyl, carboxyl, trifluoromethyl, trifluoromethoxy or straight-chain or branched alkyl, alkoxy or alkoxycarbonyl having in each case up to 8 carbon atoms, |
| $R^2$ | represents hydrogen or represents straight-chain or branched alkyl having up to 8 carbon atoms, and |
| $R^3$ | represents hydrogen or straight-chain or branched alkyl having up to 8 carbon atoms, |

and salts thereof,
characterized in that glyoxylic esters of the general formula (II)

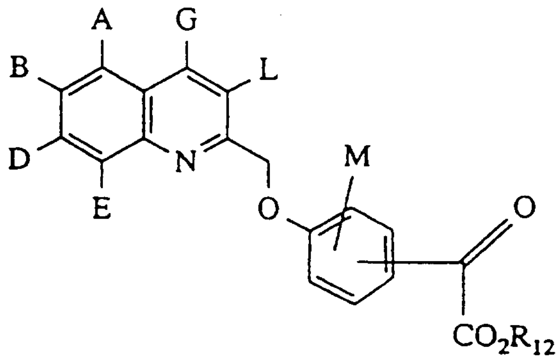

(II)

in which

A, B, D, E, G, L and M have the abovementioned meaning

and

$R^{12}$ has the abovementioned meaning of $R^3$, but does not represent hydrogen,

are reduced with Grignard or organometallic compounds of the general formula (III)

$R^1$-V (III)

in which

$R^1$ has the abovementioned meaning

and

V represents the typical Grignard radical W-Z,
wherein

W denotes magnesium, cadmium or zinc
and

Z denotes chlorine, bromine or iodine,
or
represents lithium, sodium, magnesium, aluminium, cadmium or zinc,

in inert solvents, the group V being split off,

and in the case where $R^2$ does not represent hydrogen, the products are etherified by the customary method,

and in the case of the acids ($R^3$ = H), the esters are hydrolysed,

and in the case of the enantiomers, the corresponding enantiomerically pure acids $R^3$ = H are separated by the customary methods, it being possible for the substituents A, B, D, E, G, L and M to be varied by customary methods, if appropriate.

**Revendications**

**Revendications pour les Etats contractants suivants : AT, BE, CH, DE, DK, FR, GB, GR, IE, IT, LI, LU, MC, NL, PT, SE**

**1.** Dérives substitués d'acide mandélique de formule générale (I)

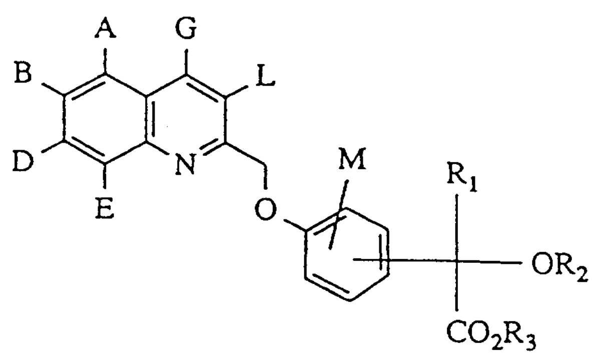

(I),

dans laquelle

A, B, D, E, G, L et M sont égaux ou différents et représentent de l'hydrogène, un groupe hydroxy, un halogène, un groupe trifluorométhyle, trifluorométhoxy ou carboxy, un groupe alkyle linéaire ou ramifié ayant jusqu'à 10 atomes de carbone, qui est substitué le cas échéant par un radical hydroxy ou un halogène, un groupe alkoxy ou alkoxycarbonyle linéaire ou ramifié ayant jusqu'à 10 atomes de carbone, ou un groupe aryle de 6 à 10 atomes de carbone, qui est substitué le cas échéant par un halogène, un radical nitro, cyano ou par un radical alkyle ou alkoxy linéaire ou ramifié ayant chacun jusqu'à 8 atomes de carbone,

$R^1$ est un groupe de formule

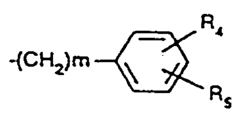
,
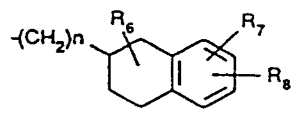
ou
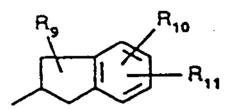

n et m sont égaux ou différents et représentent le nombre 1, 2, 3, 4, 5, 6, 7 ou 8,

$R^4$, $R^5$, $R^6$, $R^7$, $R^8$, $R^9$, $R^{10}$ et $R^{11}$ sont égaux ou différents et représentent de l'hydrogène, un halogène, un groupe nitro, cyano, hydroxy, carboxy, trifluorométhyle, trifluorométhoxy ou un groupe alkyle, alkoxy ou alkoxycarbonyle linéaire ou ramifié ayant chacun jusqu'à 8 atomes de carbone,

$R^2$ est de l'hydrogène ou un groupe alkyle linéaire ou ramifié ayant jusqu'à 8 atomes de carbone,

$R^3$ est de l'hydrogène ou un groupe alkyle linéaire ou ramifie ayant jusqu'à 8 atomes de carbone,

et leurs sels.

**2.** Dérivés substitués d'acide mandélique suivant la revendication 1, dans lesquels

A, B, D, E, G, L et M sont égaux ou différents et représentent de l'hydrogène, du fluor, du chlore, du brome, un groupe trifluorométhoxy ou carboxy, un groupe alkyle linéaire ou ramifié ayant jusqu'à 8 atomes de carbone, qui est substitué le cas échéant par un radical hydroxy, du fluor, du chlore ou du brome, un groupe alkoxy ou alkoxycarbonyle linéaire ou ramifié ayant chacun jusqu'à 8 atomes de carbone, ou bien un groupe phényle qui est substitué le cas échéant par du fluor, du chlore, du brome, un radical nitro, cyano ou par un radical alkyle ou alkoxy linéaire ou ramifié ayant chacun jusqu'à 6 atomes de carbone,

$R^1$ est un groupe de formule

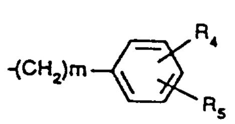
,
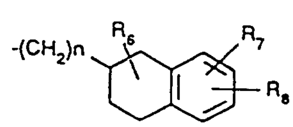
ou
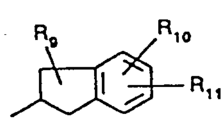

dans laquelle

n et m sont égaux ou différents et représentent le nombre 1, 2, 3, 4, 5, 6 ou 7,

$R^4$, $R^5$, $R^6$, $R^7$, $R^8$, $R^9$, $R^{10}$ et $R^{11}$ sont égaux ou différents et représentent de l'hydrogène, du fluor, du chlore, du brome, un groupe trifluorométhyle, trifluorométhoxy ou un groupe alkyle ou alkoxy linéaire ou ramifié ayant chacun jusqu'à 6 atomes de carbone,

$R^2$ est de l'hydrogène ou un groupe alkyle linéaire ou ramifié ayant jusqu'à 6 atomes de carbone,

$R^3$ est de l'hydrogène, un groupe alkyle linéaire ou ramifié ayant jusqu'à 6 atomes de carbone,

et leurs sels.

**3.** Dérivés substitués d'acide mandélique suivant la revendication 1, dans lesquels

A, B, D, E, G, L et M sont égaux ou différents et représentent de l'hydrogène, du fluor, du chlore, un groupe alkyle ou alkoxy linéaire ou ramifié ayant chacun jusqu'à 6 atomes de carbone,

$R^1$ est un groupe de formule

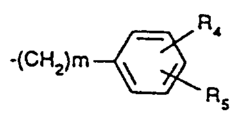 , 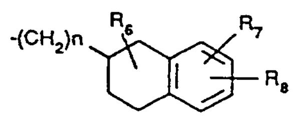 ou 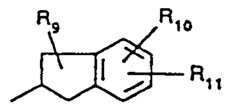

dans laquelle

n et m sont égaux ou différents et représentent le nombre 1, 2, 3, 4, 5 ou 6,

$R^4$, $R^5$, $R^6$, $R^7$, $R^8$, $R^9$, $R^{10}$ et $R^{11}$ sont égaux ou différents et représentent de l'hydrogène, du fluor, du chlore, du brome, un groupe trifluorométhyle, trifluorométhoxy ou un groupe alkyle ou alkoxy linéaire ou ramifié ayant chacun jusqu'à 4 atomes de carbone,

$R^2$ est de l'hydrogène ou un groupe alkyle linéaire ou ramifié ayant jusqu'à 4 atomes de carbone,

$R^3$ est de l'hydrogène, un groupe alkyle linéaire ou ramifié ayant jusqu'à 4 atomes de carbone,

et leurs sels.

**4.** Dérivés substitués d'acide mandélique suivant la revendication 1, dans lesquels le reste de formule $-C-(R^1)(OR^2)$ est en position 4 par rapport au reste quinolylméthoxy.

**5.** L'acide alpha-[4-(2-quinolynylméthoxy)-phényl]-alpha-hydroxy-2-indane-acétique de formule

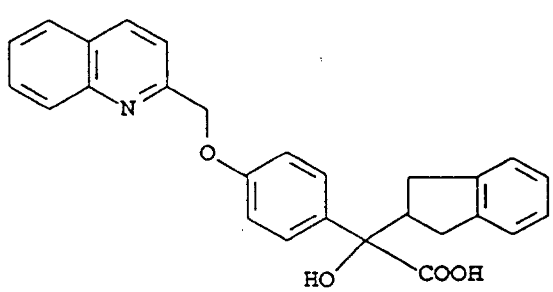

ses stéréoisomères et ses sels.

**6.** Dérivés substitués d'acide mandélique suivant les revendications 1 à 5, destinés au traitement de maladies.

**7.** Procédé de production de dérivés substitués d'acide mandélique suivant les revendications 1 à 5, caractérisé en ce qu'on réduit des esters de glyoxyle de formule générale (II)

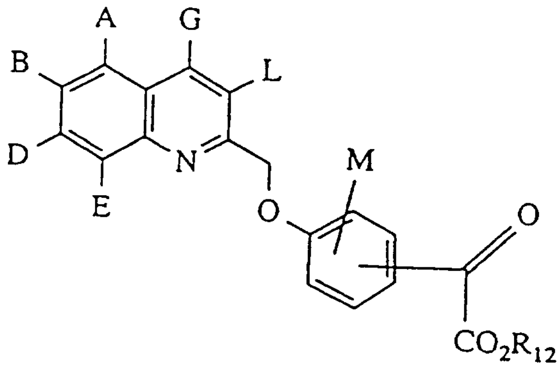

(II),

dans laquelle

A, B, D, E, G, L et M ont la définition indiquée ci-dessus

et

$R^{12}$ a la définition donnée ci-dessus pour $R^3$, mais ne représente pas un atome d'hydrogène,

avec des composés de Grignard ou des composés organométalliques de formule générale (III)

$R^1$-V (III)

dans laquelle

$R^1$ a la définition indiquée ci-dessus

et

V est un reste typique de Grignard W-Z,

dans lequel

W désigne du magnésium, du cadmium ou du zinc

et

Z désigne du chlore, du brome ou de l'iode,

ou

représente du lithium, du sodium, du magnésium, de l'aluminium, du cadmium ou du zinc

dans des solvants inertes avec élimination du groupe V,

et au cas où $R^2$ ne représente pas de l'hydrogène,

on l'éthérifie par un procédé classique,

et dans le cas des acides ($R^3$ = H) on saponifie les esters,

et dans le cas des énantiomères, on sépare les acides correspondants de pureté énantiomérique, $R^3$ = H, par les procédés classiques, et on peut alors faire varier les substituants A, B, D, E, G, L et M le cas échéant par des procédés classiques.

**8.** Médicaments contenant des dérivés substitués d'acide mandélique suivant les revendications 1 à 5.

**9.** Utilisation de dérivés substitués d'acide mandélique suivant les revendications 1 à 5 pour la préparation de médicaments.

**10.** Utilisation de dérivés substitués d'acide mandélique suivant les revendications 1 à 5 pour la production d'inhibiteurs de synthèse des leucotriènes.

**Revendication pour l'Etat contractant suivant : ES**

**1.** Procédé de production de dérivés substitués d'acide mandélique de formule générale (I)

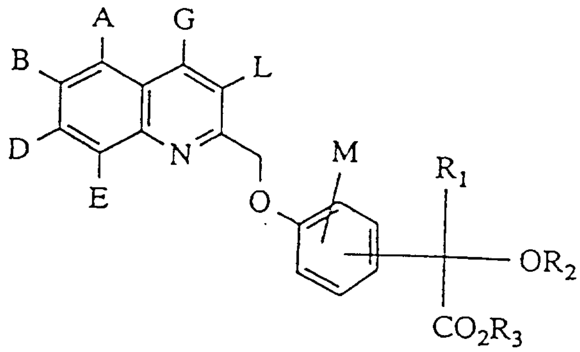

(I),

dans laquelle

A, B, D, E, G, L et M sont égaux ou différents et représentent de l'hydrogène, un groupe hydroxy, un halogène, un groupe trifluorométhyle, trifluorométhoxy ou carboxy, un groupe alkyle linéaire ou ramifié ayant jusqu'à 10 atomes de carbone, qui est substitué le cas échéant par un radical hydroxy ou un halogène, un groupe alkoxy ou alkoxycarbonyle linéaire ou ramifié ayant jusqu'à 10 atomes de carbone, ou un groupe aryle de 6 à 10 atomes de carbone, qui est substitué le cas échéant par un halogène, un radical nitro, cyano ou par un radical alkyle ou alkoxy linéaire ou ramifié ayant chacun jusqu'à 8 atomes de carbone,

$R^1$ est un groupe de formule

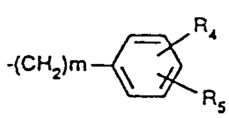

,

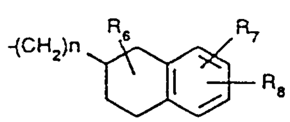

ou

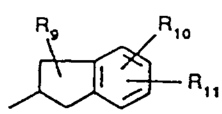

dans laquelle

n et m sont égaux ou différents et représentent le nombre 1, 2, 3, 4, 5, 6 7 ou 8,

$R^4$, $R^5$, $R^6$, $R^7$, $R^8$, $R^9$, $R^{10}$ et $R^{11}$ sont égaux ou différents et représentent de l'hydrogène, un halogène, un groupe nitro, cyano, hydroxy, carboxy, trifluorométhyle, trifluorométhoxy ou un groupe alkyle, alkoxy ou alkoxycarbonyle linéaire ou ramifié ayant chacun jusqu'à 8 atomes de carbone,

$R^2$ est de l'hydrogène ou un groupe alkyle linéaire ou ramifié ayant jusqu'à 8 atomes de carbone,

$R^3$ est de l'hydrogène ou un groupe alkyle linéaire ou ramifié ayant jusqu'à 8 atomes de carbone,

et de leurs sels, caractérisé en ce qu'on réduit des esters de glyoxyle de formule générale (II)

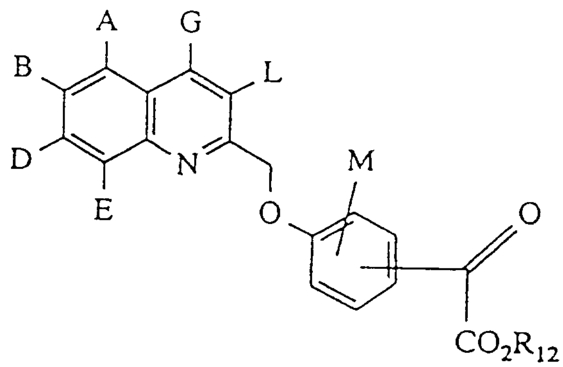

(II),

dans laquelle

A, B, D, E, G, L et M ont la définition indiquée ci-dessus

et

$R^{12}$ a la définition donnée ci-dessus pour $R^3$, mais ne représente pas un atome d'hydrogène,

avec des composés de Grignard ou des composés organométalliques de formule générale (III)

$R^1$-V (III)

dans laquelle

$R^1$ a la définition indiquée ci-dessus

et

V est un reste typique de Grignard W-Z,

dans lequel

W désigne du magnésium, du cadmium ou du zinc

et

Z désigne du chlore, du brome ou de l'iode,

ou

représente du lithium, du sodium, du magnésium, de l'aluminium, du cadmium ou du zinc

dans des solvants inertes avec élimination du groupe V,

et au cas où $R^2$ ne représente pas de l'hydrogène,

on l'éthérifie par un procédé classique,

et dans le cas des acides ($R^3$ = H) on saponifie les esters, et dans le cas des énantiomères, on sépare les acides correspondants de pureté énantiomérique, $R^3$ = H, par les procédés classiques, et on peut alors faire varier les substituants A, B, D, E, G, L et M le cas échéant par des procédés classiques.